# EUROPEAN PATENT APPLICATION

(11) **EP 3 502 167 A1**
(43) Date of publication of application: **26.06.2019**
(21) Application number: 18151287.2
(22) Date of filing: 11.01.2018
(51) Int. Cl.: C08J 3/075, A61K 47/34, A61K 47/36, C08J 3/24, C08G 63/08, C08G 63/91, C08G 65/48, C08L 5/08, C08L 67/04, C08L 71/02

(54) **A HYDROGEL AND PREPARATION METHOD THEREOF**

(30) Priority: 19.12.2017 US 201715847036
(71) Applicant: Shenzhen PromeKan Materials Inc., 518031 Shenzhen (CN)
(72) Inventor: TANG, Jian, Shenzhen, 518031 (CN)
(74) Representative: Distefano, Luigi Roberto

(57) **Abstract**

The invention provides a hydrogel comprising an aqueous chitosan solution and a water-soluble linker agent. The linker agent is a block copolymer composing non-degradable block such as poly(ethylene glycol) and hydrolytic degradable block such as poly(amino acid) and polyester. Both ends of the block copolymer are terminated with active group such as glyoxylic acid, uronic acid and aldehyde benzoic acid. When the two main components are mixed together, the resulting solution will undergo spontaneous gelation at mild condition. The resulting hydrogel is able to retain the stability of biopharmaceuticals fully, therefore, which is qualified to serve as drug vehicles used *in vivo* and topical applications.

## Description

### FIELD OF INVENTION

The present invention relates to a novel hydrogel which is qualified to be used as biomedical materials in drug vehicles and medical devices.

### BACKGROUND OF THE INVENTION

It has been a long time since some proteins were recognized to be of therapeutic effects, for example, anti-serum was used to treat diphtheria in 19th century, and insulin had been extracted from bovine and pig's pancreases to remedy patients suffering from diabetes at early 20th century. However, due to the limitations for proteins of human being sources and animal sources, such as low yield, cross virus infection and immune response, the natural pharmaceutical proteins didn't get wide applications in clinic until the emerging of gene recombination proteins. In the year of 1982 the recombinant insulin was officially approved to market America, which also marked a starting point of the practical usage for the new type of recombinant therapeutic proteins.

According to therapeutic effects, pharmaceutical proteins may be classified into four types: enzymatic or signal pathways activation, special targeting binding, vaccines and diagnosis. The first two types are basically pharmaceutical proteins. To date more than one hundred of pharmaceutical proteins have been marketing in the world and most of which are recombinant proteins.

Although there is a bright future of development and usage for pharmaceutical proteins, one special weakness for proteins is that the three-dimensional structure of protein molecular is vulnerable and sensitive to the surrounding physical and chemical environments. Once the proteins happen deconstructing, they will lose bioactivities. Thus, therapeutic proteins are currently almost administrated through intravenous injection and also require a very careful condition to keep their bioactivities in the process of producing, shipping and storing.

In addition to the above mentioned shortage, a lot of proteins present a short half-time in blood circulation, which directly results in frequent injections in one day or one week. Severely, high frequency of injection brings to patients great mental stress and low life quality. Alternative administration routes except intravenous injection, or other dosage forms are summoned in clinic, such as long-term acting formulations which can reduce injections, or nasal, oral, pulmonic administration, or topical application.

In order to elongate the retention time of protein in blood circulation, various methods have been investigated. Representatively, liposome or phospholipids micelle are common carriers used to enclose protein and administered through intravenous injection. One shortage, however for this type of micro-carrier is that accumulation of lipids will increase the level of blood lipids harmfully.

Also, there are reports that many biomedical materials such as collagen, synthetic PLA, PLGA have been attempted to develop in micro- or nano-carriers for proteins. Yet their rude preparation methods involving the usage of organic solvents, rough agitation, heating and pH change will damage the integrity of molecular structure of protein. Moreover, plenty of protein will loss in the preparation process, which will terribly increase the manufacturing cost of medicine.

Protein molecular modification is another method aiming to increase the maintenance time for pharmaceutical protein in the blood circulation, whose principle is that by covalent binding inert molecular to therapeutic protein, the resulting complex protein will change its original membrane permeability or enzymatic restriction sites. Take PEGylation as an example, in this technique a big PEG(polyethylene glycol) molecular covalently binds to a protein. The PEG molecular can shield the enzymatic restriction sites of protein and therefore the pharmaceutical protein won't be quickly digested by enzyme in the blood circulation. But big challenges for this crafts are to how to keep a batch of resulting complex protein homogenously and to retain the therapeutic activity of protein very well.

Besides the medicine, in tissue engineering area scaffold is often considered to guide cells to grow into a functional tissue. Inside the scaffold essential growth factors are encapsulated and will be sustainably released out to induce the cell growth and differentiation. Common materials applied to build a scaffold involve animal source of collagen, natural chitosan, cellulose, alginates and polyphosphate salts, synthetic PLGA, PLA, etc. But rough methods are also employed in the preparation. Especially, when PLGA and PLA hydrolytically degrade, the resulting acids will accumulate to change the pH value of the local site, which will destroy the growth factors and cause local inflammation also.

Hydrogel is one type of friendly vehicle for therapeutic proteins. Basically hydrogel is cross-linked hydrophilic polymer and its network can retain large amounts of water. Macromolecule protein can be restrained in hydrogel's three-dimensional network while remaining integral molecular structure. Hydrogel is also able to be injected into body, which doesn't require extra surgery. Several types of hydrogel have been reported to serve as carriers for therapeutic proteins. One type is high viscosity of hydrogel making up of alone collagen, chitosan, cellulose, PLGA solution, or their combinations. There are no physical or chemical crosslinks in the solutions, so the hydrogel will disperse in a short time. Another type is thermosensitive hydrogel, which presents sol situation in room temperature and then becomes in gel after being injected into body. But this type of hydrogel usually composes of a lot of acid polymer such as PLA, PLGA, its degradation products led to acid accumulation which is going to cause local inflammation. The third type is to use toxic agent such as paraformaldehyde, glyoxal and glutaraldehyde to cross-link chitosan into hydrogel. These hazardous aldehydes are harmful to health and difficult to be removed completely.

In summary, there is still a great room to improve the specifications of hydrogel to adjust it to suit for protein vehicle. This present invention is committed to develop a bio-friendly hydrogel which can retain the activity of therapeutic protein very well while possessing practical value in clinic applications and pharmaceutical industry.

### DESCRIPTION OF THE INVENTION

This present invention intends to provide a novel hydrogel which covers the following advantages:
- injectable, coatable and biodegradable
- retain the activity of therapeutic protein fully
- excellent biocompatibility

In order to achieve the strong points described above, a hydrogel is gelated at mild condition, namely, in aqueous solution with pH value in the range of 4.5 ∼ 9.0 and temperature in the range of 0 - 60 °C. The pre-hydrogel solution is able to be spontaneous gelation, no extra chemical catalytic or photocatalytic, or thermal catalytic, or acid or base catalytic requirements.

The hydrogel contains two basic components: soluble chitosan and linker agent. The molecular weight of chitosan is from about 50,000 to 500,000 Da. The solvent used to prepare chitosan and linker agent solution is selected from the group consisting of pure water, saline solution, cell culture media, phosphate buffer, dilute acid and alkaline.

The linker agent is a block copolymer which involves two types of block, non-degradable block (represented by "A") and degradable block (represented by "B"). The two types of block are arranged in orders of AB, or ABA, or BAB. Inside a linker agent, the molecular weight of "A" accounts for from 50% to 90% of the total molecular weight and "B" takes up the rest molecular weight from 10% to 50%. Non-degradable block is preferable PEG(polyethylene glycol) whose molecular weight is from 300 to 20,000 Da. Degradable block is selected from a group consisting of poly-amino-acid, polyglycolide, polylactide, polyglycide-lactide, polycaprolactone, and combinations thereof. At both ends of block copolymer are covalently linked aldehyde groups, and the active groups is selected among glyoxylic acid, uronic acid, aldehyde benzoic acid, and combinations thereof.

In the hydrogel, chitosan accounts for a portion of mass in a range of from 0.1% to 30% and linker agent takes up a portion of mass in a range of from 0.1% to 90%. The gelling time is adjustable in a range of from 1 to 100 minutes. The viscosity of the hydrogel is adjustable in a range of from 0.1 to 10 Pa•s.

Summarily, this invention provides a hydrogel which contains chitosan and linker agent. Chitosan is aqueous solution, which takes up from 0.1% to 30% of total mass of a hydrogel. The linker agent is aqueous solution too, taking up from 0.1% to 90% of total mass of a hydrogel. The linker agent is a block copolymer consisting of two types of polymers: non-degradable polymer taking up from 50% to 90% of total molecular weight of the copolymer, and degradable polymer occupies from 10% to 50% of total molecular weight of the copolymer. Non-degradable polymer is polyethylene glycol. Degradable polymer is selected from a group consisting of poly amino acid, polyglycolide, polylactide, polyglycide-lactide, polycaprolactone, and combinations thereof. The arranged arrays of non-degradable block (represented by "A") and degradable block (represented with "B") in the block copolymer includes AB, ABA and BAB. The block compolyer are terminated with active groups which are selected from a group consisting of glyoxylic acid, uronic acid, aldehyde benzoic acid, and combinations thereof.

Further, chitosan refers to that type of chitosan which has 50% to 90% of deacetylation, chitosan oligosaccharides, chitosan sulfate, chitosan hydrochloride, Chitosan lactate, carboxymethyl chitosan, glycol chitosan, and combinations thereof.

Further, poly(ethylene glycol) has a molecular weight from 300 to 20,000 Da. Polyglycolide has a molecular weight from 500 to 20,000 Da. Polylactide has a molecular weight from 300 to 20,000 Da. Polyglycolide lactide(PLGA) has a molecular weight from 3,000 to 20,000 Da. Polycaprolactone have a molecular weight from 3,000 to 20,000 Da. In the molecule of polyglycide lactide, the molecular weight ratios of polylactide to polyglycolide include 50:50, 65:35, 75:25 and 85:15.

This invention provides one method to prepare the hydrogel:
Step 1: Poly(ethylene glycol), whose both ends are hydroxyl groups, was heated to 120 ∼ 160°C under nitrogen protection. Degradable polymer and catalyst I were added to the poly(ethylene glycol). 6 ∼ 8 hours later, insoluble substance was collected and dried at 20 - 35°C to attain a constant weight, then named as "A". Alternatively, poly(ethylene glycol) was dissolved in dichloromethane or in tetrahydrofuran. Degradable polymer and catalyst II were added to the poly(ethylene glycol) solution. 5 ∼ 60 minutes later, the outcome was precipitated with ether and then experienced dialysis and lyophilization. The dried product was named as "B". Herein the degradable polymer is selected from a group consisting of poly amino acid, polyglycolide, polylactide, polyglycolide lactide, polycaprolactone, and combinations thereof. In the block compolymer the molecular weight of degradable block takes up a range from 10% to 50% of total molecular weight. Catalyst I is stannous octoate. Catalyst II is 1,8-Diazabicyclo[5.4.0]undec-7-ene(DBU).
Step 2: The intermediate substance "A" or "B" was dissolved in organic solvent. Chemical "C" was added to the "A" or "B" solution and the mole amount of "C" is from 2 to 10 times greater than "A" or "B". Catalyst III was added to the resulting solution. 24 - 48 hours later, the final outcome, linker agent was concentrated. Herein Organic solvent is selected from ethanol, dichloromethane(DCM) and tetrahydrofuran. "C" is selected from a group consisting of glyoxylic acid, uronic acid and aldehyde benzoic acid. Catalyst III includes 4-Dimethylaminopyridine(DMAP), N-(3-Dimethylaminopropyl)-N'-ethylcarbodiimide hydrochloride(EDC) or N,N'-Dicyclohexylcarbodiimide.

Further, Glyoxylic acid refers to D-glucuronic acid, D-galacturonic acid or D-mannuronic acid. Aldehyde benzoic acid refers to O-aldehyde benzoic acid, m-aldehyde benzoic acid or p-aldehyde benzoic acid.

Step 3: Chitosan and linker agent were dissolved in aqueous solutions respectively. Appropriate amount of chitosan solution and linker agent solution were mixed together according to the final concentrations(m/v) in the resulting solution: chitosan 0.1% ∼ 30%, linker agent 0.1%∼90%.

Further, the viscosity of the hydrogel is in a range from 0.1 to 10 Pa•s.

This invention also provides another method to prepare the hydrogel:
Step 1: mPEG(methoxypolyethylene glycol) was dehydrated in 120 ∼ 160°C under nitrogen protection. Degradable polymer and catalyst I were added to the mPEG. 6 ∼ 8 hours later, insoluble substance was collected and dried at 20 ∼ 35°C, then named as "A". Alternatively, mPEG was dissolved in organic solvent. Degradable polymer and catalyst II were added to the mPEG solution. 5 ∼ 60 minutes later, ether was added to the resulting solution, then the precipitate was collected and named as "B". The degradable polymer is selected from a group consisting of poly amino acid, polyglycolide, polylactide, polyglycolide lactide), polycaprolactone, and combinations thereof. Inside the molecular of "A" and "B", the molecular weight of degradable block takes up from 10% to 50% of the total molecular weight of the copolymer. The catalyst I is stannous octoate and catalyst II is 1,8-Diazabicyclo[5.4.0]undec-7-ene(DBU). The organic solvent is dichloromethane(DCM) or tetrahydrofuran.
Step 2: The intermediate product "A" or "B" was dissolved in sulfur ethanol. Catalyst III was added to the solution and the resulting solution was stirred for 6 ∼ 24 hours. The outcome was concentrated and named as "C". Alternatively, the product "A" or "B" was dissolved in dichloromethane. Catalyst IV was added to the solution and the resulting solution was refluxed at 100 ∼ 130°C for 2 ∼ 12 hours. The outcome was concentrated and named as "D". The catalyst III is Aluminum chloride (AlCl₃) and catalyst IV is hydrogen iodide;
Step 3: The intermediate outcome "C" or "D" was dissolved in organic solvent. Chemical "E" and catalyst V were added to the "C" or "D" solution. The molar amount of "E" was from 2 to 10 times greater than "C" and "D". 24 ∼ 48 hours later, the final outcome, linker agent was concentrated. Herein, the organic solvent is selected from ethanol, dichloromethane(DCM) and tetrahydrofuran. The chemical "E" is selected from glyoxylic acid, uronic acid and aldehyde benzoic acid. The catalyst V includes 4-Dimethylaminopyridine(DMAP), N-(3-Dimethylaminopropyl)-N'-ethylcarbodiimide hydrochloride(EDC) or N,N'-Dicyclohexylcarbodiimide.

Further, the uronic acid is selected from D-glucuronic acid, D-galacturonic acid and D-mannuronic acid. The aldehyde benzoic acid is selected from O-aldehyde benzoic acid, m-aldehyde benzoic acid and p-aldehyde benzoic acid.

Step 4: Chitosan and the synthetic linker agent were dissolved in aqueous solution separately. Appropriate amount of the two stock solutions were mixed in according to the final concentrations(m/v) in the resulting solution: chitosan 0.1% ∼ 30%, linker agent 0.1% ∼ 90%.

Further, the viscosity of the hydrogel is in a range from 0.1 to 10 Pa•s.

The hydrogel provided in the present invention can serve as vehicles for biopharmaceuticals and other bioactive substances, and also can be injected or implanted *in vivo.* The hydrogel's degradation rate is adjustable in a range from 1 to 180 days. In addition, the hydrogel can also be applied topically.

The loading amount of drug in the hydrogel provided in the present invention:
(1) For water-soluble medicine, their saturated solutions are the maximum drug loading.
(2) For low-water-soluble drugs, they can be blended with the hydrogel.

The detailed preparation method for the hydrogel provided in the present invention is following:
(1) Dilute acid, neutral or physiological pH of water or aqueous solution all can be used to prepare chitosan stock solution. The concentration(mass/volume) of chitosan stock solution is in a range from 0.1% to 30.0%.
(2) The same solvent described above was also used to make the stock solution of linker agent. The concentration of linker agent was in a range from 0.1% to 100.0%.
(3) Upon demands, appropriate amount of chitosan stock solution is mixed with medicine. Stock solution of linker agent was added to the resulting chitosan and medicine solution. The final resulting solution was put at 0 ∼ 60°C environment and would gelate within from 1 to 100 minutes.

The advantages for the hydrogel provided in the present invention include:
(1) The hydrogel not only is used *in vivo,* also is used topically;
(2) Before gelation, the pre-hydrogel solution can be injected into body instead of doing surgery;
(3) Both viscosity and degradation rate parameters of the hydrogel are adjustable, which crucially decide the release profile of drug encapsulated in the hydrogel vehicle;
(4) The gelation condition for the hydrogel is very mild, which is especially beneficial to retain the stability/activity of biopharmaceuticals;
(5) This hydrogel is of antibacterial function because one of its components, chitosan has been proved to be antibacterial and bacteriostatic effects.

Further, the main component, chitosan has been widely used in medical devices and cosmetics fields. As for the linker agent, the non-dregradable block, poly(ethylene glycol) has also been widely found in medicine as pharmacy excipient; the biodegradable block polymers have been used to make long-term acting dosage carrier and tissue engineer scaffold; the active aldehyde groups come from aromatic aldehyde or biochemical aldehyde, which present non-toxicity nature when binded at the ends of a long polymer chain. So the biocompatibility of the hydrogel is ensured definitely.

The preparation process of the hydrogel is convenient. Briefly, preparing the stock solutions of chitosan and linker agent respectively, then mixing the two stock solutions gently, next, the resulting solution will undergo a spontaneous gelation.

In all, the hydrogel provided in the present invention owns the beneficial characteristics that it is made up of biocompatible components and can be formed under a very mild condition, which is particularly beneficial to preserve the stability of biopharmaceuticals. Along with the degradation of the linker agent, the hydrogel will disintegrate gradually to release out the drugs encapsulated. The final degradation products will be cleared out through metabolizing. Due to the adjustability of viscosity and degradation rate of the hydrogel, the release profile of the loading pharmaceuticals is controllable. Moreover, the hydrogel is able to be injectable and topical application.

### Embodiment

The chitosan used for the prevent invention are commercially available from Santa Cruz Biotechnology (Dallas, USA) and from Heppe Medical Chitosan (HMC, Halle, Germany). Poly(ethylene glycol) is commercially available from Alfa aesar(Thermo Fisher Scientific, Canada). Lactide is commercially available from Sigma-Aldrich(Oakville, Canada). Catalysts are commercially available from various sources.

The core job for the present invention is the design and synthesis of linker agent. Thus, Most of the specific embodiments were referred to address the preparation of linker agent in detail.

### EXAMPLE 1: Synthesis of linker agent

0.5 g of mPEG (molecular weight 5,000 Da) was dissolved in 1 mL of DCM, and 10 mg of DBU was added to the mPEG solution. 0.8 g of lactide was dissolved in 5 mL of DCM and then mixed with the mPEG solution. The resulting solution was stirred for 10 minutes. Ether was added to the resulting solution. The precipitation was collected and named as product I. The intermediate product I was dissolved in 60 mL of thioethanol and an equi-molar amount of AlCl₃ was added to the product I solution. The resulting solution was stirred for 24 hours. DCM was used to extract the product II. The intermediate product II was dissolved in 100 mL of DCM. 5 times of molar amount of aldehyde benzoic acid and EDC and 0.5 times of molar amount of DMAP were added to the product II solution. The resulting solution was stirred for 24 hours. Ether was added to the resulting solution. The precipitation was collected, which was the linker agent whose number average and weight average molecular weight (Mn, Mw) were 10619 and 10848 respectively and polydispersity index was (Mw / Mn) 1.02.

### EXAMPLE 2: Synthesis of linker agent

1g of poly(ethylene glycol) (molecular weight 2,000 Da) was dissolved in 5 mL of DCM and 10 mg of DBU was added to the PEG solution. 0.5 g of lactide was dissolved in 3 mL of DCM and then mixed with the PEG solution. The resulting solution was stirred for 10 minutes. Ether was added to the resulting solution. The precipitation was collected and named as product I. The intermediate product I was dissolved in 50 mL DCM. 5 times of molar amount of aldehyde benzoic acid and EDC and 0.5 times of molar amount of DMAP were added to the product I solution. The resulting solution was stirred for 24hours. The outcome was concentrated, which was the final linker agent. ¹H NMR confirmed the molecular weight ratio of PEG to polylactide(PLA) in the copolymer was about 1: 0.5.

### EXAMPLE 3: Synthesis of linker agent

1 g poly(ethylene glycol) (molecular weight 2,000 Da) was dried at 120°C under vacuum condition, then increased until 160°C in nitrogen atmosphere. 0.5 g of lactide and 0.2%(mass/mass) stannous octoate were added to the poly(ethylene glycol). 8 hours later, the insoluble material was collected. The dried outcome was product I. Inside the molecular of product I the molecular weight ratio of poly(ethylene glycol) to polylactide was about 1: 0.5 confirmed by ¹H NMR.

The intermediate product I was dissolved in 50 mL of DCM. 5 times of molar amount of aldehyde benzoic acid and EDC and 0.5 times of molar amount of DMAP were added to the product I solution. The resulting solution was stirred for 24 hours. The final outcome was concentrated, which was the linker agent.

### EXAMPLE 4: Preparation of blank hydrogel

2 g of glycol chitosan was dissolved in 100 mL water. 0.25 g of linker agent was dissolved in 1 mL of water. 500 µL chitosan solution, 480 µ water and 20 µL linker agent solution were mixed together gently. The resulting solution underwent spontaneous gelation in 5 minutes.

### EXAMPLE 5: Preparation of hydrogel loading protein

1 g of bovine serum albumin was dissolved in 10 mL phosphate buffer (pH 7.2-7.6). 500 µL chitosan solution(described in example 4), 100 µL bovine serum albumin solution, 380 µL water and 20 µL linker agent solution(described in example 4) were mixed together gently. The resulting solution underwent spontaneous gelation in 5 minutes.

The above-described examples are several specific embodiments for the present invention but do not limit the scope of the practical application of the invention. Any equivalent transformations based on the description of the present invention, or usages directly or indirectly in relational fields, were within the scope of patent protection of the present invention.

## Claims

1. A hydrogel, comprising 0.1% ∼ 30% (mass/volume) of chitosan, 0.1% ∼ 90% (mass/volume) of linker agent and aqueous solution, wherein said the linker agent is a block copolymer whose both ends are terminated with active group. Wherein said the block copolymer comprises 50% ∼ 90% (molecular weight) of non-degradable polymer(represented by "A") and 1% ∼ 50% (molecular weight) of hydrolytic biodegradable polymer(represented by "B"), wherein said polymer "A" is preferably poly(ethylene glycol), and said polymer "B" is selected from a group consisting of poly-amino-acids, polyglycolide, polylactide, polylactide glycolide, polycaprolactone, and combinations thereof, wherein said the arrangement orders of polymer "A" and "B" in the block copolymer include AB, ABA and BAB. The active group said is selected from a group consisting of glyoxylic acid, uronic acid, aldehyde benzoic acid, and combinations thereof.

2. A hydrogel according to claim 1, wherein said chitosan is selected from a group consisting of Chitosan, chitosan oligosaccharides, chitosan sulfate, chitosan hydrochloride, chitosan lactate, carboxymethyl chitosan, hydroxyethyl chitosan, and combinations thereof, wherein said chitosan has degree of deacetylation from 50% to 90%.

3. A hydrogel according to claim 1, wherein said poly(ethylene glycol) has a molecular weight from 300 to 20,000 Da, and said polyglycolide has a molecular weight from 500 to 20,000 Da, and said polylactide has a molecular weight from 300 to 20,000 Da, and said polycaprolactone has a molecular weight from 3,000 to 20,000 Da, and said polylactide glycolide has a molecular weight from 3,000 to 20,000 Da, wherein said the molecular weight ratios of polylactide to polyglycolide in polylactide glycolide contain 50:50, 65:35, 75:25 and 85:15.

4. A hydrogel according to claim 1, wherein said the preparation method of the said hydrogel is following:
Step 1: Poly(ethylene glycol) which is both terminated with hydroxyl group is dehydrated at 120~160°C and vacuum environment. Degradable polymer and catalyst I are added to the poly(ethylene glycol) under nitrogen protection. 6 ∼ 8hours later, the insoluble substance is collected and dried at 20 ∼ 35°C until keeping constant weight. The dried substance is named as "A". Alternatively, poly(ethylene glycol) is dissolved in dichloromethane or in tetrahydrofuran. Degradable polymer and catalyst II are added to poly(ehtylene glycol) solution. 5 ∼ 60 minutes later, ether is added to the resulting solution. The precipitation is collected and named as "B", wherein said degradable polymer is selected from a group consisting of poly amino acid, polyglycolide, polylactide, polyglycolide lactide, polycaprolactone, and combinations thereof, and said the molecular weight ratio of poly(ethylene glycol) to biodegradable polymer is from 2 to 10, and said catalyst I is stannous octoate, and said catalyst II is 1,8-Diazabicyclo(5.4.0)undec-7-ene;
Step 2: The intermediate outcome "A" or "B" is dissolved in organic solvent. Chemical "C" and catalyst III are added to "A" or "B" solution. 24 ∼ 48 hours later, the outcome is linker agent. Wherein said organic solvent is selected from a group consisting of ethanol, dichloromethane and tetrahydrofuran, and said chemical "C" is selected from a group consisting of glyoxylic acid, uronic acid, aldehyde benzoic acid, and combinations thereof, and said the mole ratio of both "C" to "A" and "C" to "B" are from 2 to 5, said catalyst III are combination of 4-Dimethylaminopyridine and (3-Dimethylaminopropyl)-N'-ethylcarbodiimide hydrochloride, or combination of 4-Dimethylaminopyridine and N,N'-Dicyclohexylcarbodiimide;
Step 3: Chitosan and linker agent are dissolved aqueous solution separately. The two stock solutions are mixed together at 0 ∼ 60°C, then the mixed solution will gelate in from 1 to 90 minutes, wherein said the aqueous solution has a pH value from 4 to 10, said the mass/volume ratio of chitosan in the mixed solution is from 0.1% to 30% and of linker agent from 0.1% to 90%.

5. A hydrogel according to claim 4, wherein said uronic acid is selected from a group consisting of D-glucuronic acid, D-galacturonic acid, D-mannuronic acid, and combinations thereof, and said aldehyde benzoic acid is selected from a group consisting of O-aldehyde benzoic acid, m-aldehyde benzoic acid, p-aldehyde benzoic acid, and combinations thereof.

6. A hydrogel according to claim 4, wherein said the hydrogel has a viscosity in a range from 0.1 to 10 Pa•s.

7. A hydrogel according to claim 1, wherein said the preparation method of the said hydrogel is following:
Step 1: Methoxyl Poly(ethylene glycol) is dried at 120 ∼ 160°C under vacuum environment. Degradable polymer and catalyst I are added to the methoxyl poly(ethylene glycol) under nitrogen protection. 6 ∼ 8hours later, insoluble matter is collected, dried at 20 ∼ 35°C and named as "A". Alternatively, methoxyl poly(ethylene glycol) is dissolved in organic solvent. Degradable polymer and catalyst II are added to the methoxyl poly(ethylene glycol) solution. 5 ∼ 60 minutes later, ether is added to the resulting solution. The precipitation is collected and named as "B", wherein said degradable polymer is selected from a group consisting of poly amino acid, polyglycolide, polylactide, polyglycolide lactide, polycaprolactone, and combinations thereof, and said the molecular weight ratio of poly(ethylene glycol) to biodegradable polymer is from 5 to 10, and said catalyst I is stannous octoate, and said organic solvent is dichloromethane or tetrahydrofuran, and said catalyst II is 1,8-Diazabicyclo(5.4.0)undec-7-ene;
Step 2: The intermediate outcome "A" or "B" is dissolved in sulfur ethanol. Catalyst III is added to the "A" or "B" solution. 6 ~ 24 hours later, the outcome is concentrated and named as "C". Alternatively, the intermediate outcome "A" or "B" is dissolved in organic solvent. Catalyst IV is added to the "A" or "B" solution and the resulting solution is refluxed at 60 ∼ 130°C for 2 ∼ 12 hours. The outcome is concentrated and named as "D", wherein said catalyst III is aluminum chloride, and said organic solvent is dichloromethane or tetrahydrofuran, and said catalyst IV is hydrogen iodide;
Step 3: The intermediate outcome "C" or "D" is dissolved in organic solvent. Chemical "E" and catalyst V are added to the "A" or "B" solution. 24 ∼ 48 hours later, the outcome, linker agent is concentrated, wherein said organic solvent is selected from a group consisting of ethanol, dichloromethane and tetrahydrofuran, and said chemical "E" is selected from a group consisting of glyoxylic acid, uronic acid, aldehyde benzoic acid, and combinations thereof, and said the mole ratios of both "E" to "C" and "E" to "D" are from 2 to 5, and said catalyst V is combination of 4-Dimethylaminopyridine and (3-Dimethylaminopropyl)-N'-ethylcarbodiimide hydrochloride, or combination of 4-Dimethylaminopyridine and N,N'-Dicyclohexylcarbodiimide;
Step 4: Chitosan and linker agent are dissolved in aqueous solution separately. The two stock solutions are mixed together at 0 ∼ 60°C, then the mixed solution will gelate in from 1 to 90 minutes, wherein said the aqueous solution is selected from a group consisting of pure water, saline solution, cell culture media, phosphate buffer, dilute acid and alkaline, whose pH values are in a range from 4 to 10, and said the mass/volume ratio of chitosan in the mixed solution is from 0.1% to 30% and of linker agent from 0.1% to 90%.

8. A hydrogel according to claim 7, wherein said uronic acid is selected from a group consisting of D-glucuronic acid, D-galacturonic acid, D-mannuronic acid, and combinations thereof, and said aldehyde benzoic acid is selected from the group consisting of O-aldehyde benzoic acid, m-aldehyde benzoic acid, p-aldehyde benzoic acid, and combinations thereof

9. A hydrogel according to claim 7, wherein said the hydrogel has a viscosity in a range from 0.1 to 10 Pa•s.

10. A hydrogel according to claim 1, wherein said the hydrogel will hydrolytically degrade in a time course from 1 day to 60 days.

11. A hydrogel according to claim 1, wherein said pharmaceutical proteins encapsulated in this hydrogel keep activity no less than 90% of initial value.
